(19) Europäisches Patentamt
European Patent Office
Office européen des brevets

(11) **EP 4 115 799 A1**

(12) **EUROPEAN PATENT APPLICATION**

(43) Date of publication:
**11.01.2023 Bulletin 2023/02**

(21) Application number: **21184845.2**

(22) Date of filing: **09.07.2021**

(51) International Patent Classification (IPC):
**A61B 5/0205** (2006.01)   **A61B 5/053** (2021.01)
**A61B 5/0535** (2021.01)   **A61B 5/02** (2006.01)
**A61B 5/347** (2021.01)   **A61B 5/00** (2006.01)
**A61N 1/37** (2006.01)   **A61B 5/346** (2021.01)

(52) Cooperative Patent Classification (CPC):
**A61B 5/02007; A61B 5/0205; A61B 5/053;
A61B 5/346; A61B 5/347; A61B 5/7257**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Terra Quantum AG**
**9400 Rorschach (CH)**

(72) Inventors:
• **Belousov, Yury**
**9400 Rorschach (CH)**

• **Elkin, Nikolay**
**9400 Rorschach (CH)**
• **Revenko, Sergey**
**9400 Rorschach (CH)**
• **Tarakanov, Igor**
**9400 Rorschach (CH)**
• **Tikhomirova, Lyudmila**
**9400 Rorschach (CH)**

(74) Representative: **Kretschmann, Dennis**
**Boehmert & Boehmert
Anwaltspartnerschaft mbB
Pettenkoferstrasse 22
80336 München (DE)**

(54) **A METHOD AND SYSTEM FOR CONVERTING PHYSIOLOGICAL SIGNALS**

(57) The disclosure relates to a method for converting physiological signals, comprising: obtaining a first signal as a function of a time parameter, wherein the first signal represents electrocardiogram data; obtaining a second signal as a function of the time parameter, wherein the second signal represents physiological data different from the electrocardiogram data; mixing the first signal and the second signal to obtain a mixed signal; and generating a frequency spectrum pertaining to the mixed signal.

Fig. 2

EP 4 115 799 A1

**Description**

Technical Field

The disclosure relates to techniques for converting physiological signals, in particular for converting physiological signals into a frequency spectrum that is amenable to analysis.

Background

An analysis of the state of living systems based on acquired electrical signals plays an important role in the context of non-invasive research and diagnosis. Electrical signals that allow examining the state of the circulatory system of the human body or animal body are one important example. In this context, a meaningful physical characteristic of the condition of blood vessels, in particular arteries, is their hydraulic impedance, whose variation may reflect different physical states of the artery. For instance, the artery can be different states after exposure to different medication. It has been shown experimentally that the artery can change its cross-section and, accordingly, its hydraulic impedance, as a result of the natural elasticity of the vessel walls (passive state), and can also be in a state with a rigid wall (active state). The hydraulic impedance may be measured directly by means of a probe placed in the flow canal of the artery. But of greater practical relevance are electrical resistance measurements along the length of the artery, which may also reflect the hydraulic impedance. Advantageously, these latter techniques provide a non-invasive way of obtaining important information about the circulatory system.

Conventionally, the variation of the hydraulic impedance over time has been determined via electrical resistance measurements along the length of the artery, and the resulting time series has been converted into a frequency spectrum by means of a Fourier transform. Other transforms, such as the Radon transform, have likewise been employed in this context, cf. Y. M. Belousov et al., "Tomographic representation of ECG signal", J. Russ. Laser Res. vol. 39 issue 3 (2018).

However, it has been found that these conventional signal conversion and analysis techniques sometimes result in spectra that hardly differ between different states of the artery, and hence fail to provide sufficiently clear distinctions between different states of the artery.

Overview

In view of these considerations and shortcomings, improved techniques for converting physiological signals that permit a clearer and more accurate distinction between different physiological states of the underlying living system are desirable.

These objectives are addressed with a method for converting physiological signals according to independent claim 1, and a system for converting physiological signals according to independent claim 11. The dependent claims relate to preferred embodiments.

In a first aspect, the disclosure relates to a method for converting physiological signals, comprising: obtaining a first signal as a function of a time parameter, wherein the first signal represents electrocardiogram data; obtaining a second signal as a function of the time parameter, wherein the second signal represents physiological data different from the electrocardiogram data; mixing the first signal and the second signal to obtain a mixed signal; and generating a frequency spectrum pertaining to the mixed signal.

Mixing the physiological data to be analyzed with electrocardiogram data, and generating a frequency spectrum of the mixed signal, may provide for a better distinguishability between different states of the living system in many practically relevant scenarios. In this context, the electrocardiogram data may provide for a frame or reference that may bring out distinctions between the different states that would sometimes escape conventional conversion and analysis techniques.

In the context of the present disclosure, the physiological data may be any data pertaining to a human body or to an animal body.

In particular, the physiological data may comprise electrical physiological data. In the context of the present disclosure, electrical physiological data may denote data that has been obtained from electrical measurements on the human body or animal body, or measurement signals acquired from the human body or animal body that have been subsequently converted into electrical signals.

The electrocardiogram (ECG) data may pertain to the same human body or to the same animal body. In particular, the electrocardiogram data may be data sampled concurrently with the physiological data.

According to an embodiment, the physiological data may comprise rheogram data.

In the context of the present disclosure, rheogram data may denote any data pertaining to a flow of a liquid or a gas.

Specifically, the physiological data may comprise rheogram data of a blood vessel, such an artery of the human body or animal body.

According to an embodiment, the physiological data may pertain to an electrical impedance and/or a hydraulic impedance, in particular to an electrical impedance and/or a hydraulic impedance of a blood vessel, such as an artery.

The first signal may be obtained over a predetermined first time interval, and/or the second signal may be obtained over a predetermined second time interval.

Optionally, the second time interval corresponds to the first time interval.

The first signal may comprise a plurality of first signal values associated with a first plurality of different points in time. Each of the first signal values may reflect an electrocardiogram measurement pertaining to the respective point in time. Optionally, the first plurality of different points in time lie within the predetermined first time interval.

Similarly, the second signal may comprise a plurality of second signal values associated with a second plurality of different points in time. Each of the second signal values may reflect a measurement of a physiological parameter or characteristic pertaining to the respective point in time, wherein the physiological parameter or characteristic does not constitute nor pertain to an electrocardiogram. Optionally, the second plurality of different points in time lie within the predetermined second time interval.

According to an embodiment, the method may comprise normalizing the first signal and/or normalizing the second signal, prior to the mixing of the first signal and the second signal.

Normalizing the first signal and/or the second signal may allow rendering the first signal and the second signal dimensionless, and may also facilitate the mixing of signal values of different types and different amplitude ranges.

Normalizing the first signal may comprise dividing the first signal by a first normalization value. For instance, normalizing the first signal may comprise dividing the first signal by a first maximum value attained by the first signal over at a predetermined first time interval.

Similarly, normalizing the second signal may comprise dividing the second signal by a second normalization value. For instance, normalizing the second signal may comprise dividing the second signal by a second maximum value attained by the second signal over a predetermined second time interval.

In particular, the second time interval may correspond to the first time interval.

In the context of the present disclosure, mixing the first signal and the second signal may involve any operation that correlates the first signal and the second signal. A mixed signal, and the context of the present disclosure, may denote any signal that depends both on the first signal and on the second signal.

According to an embodiment, mixing the first signal and the second signal comprises linearly combining first signal and the second signal. In particular, the first signal and the second signal may be combined linearly with equal weight factors.

Specifically, the mixed signal may be given in terms of a sum of the first signal and the second signal.

A linear combination of signals is relatively straightforward to implement electronically, and may require less processing and memory resources than non-linear signal combinations.

However, in other embodiments the mixed signal may comprise a non-linear combination of the first signal and the second signal, such as a quadratic combination or terms of higher order.

The mixing may comprise mixing the normalized first signal and the normalized second signal to obtain the mixed signal.

According to an embodiment, generating the frequency spectrum pertaining to the mixed signal may comprise subjecting the mixed signal to an integral transform.

The frequency spectrum may correspond to the frequency spectrum of the integral transform, or may correspond to the frequency spectrum of a function of the integral transform, such as a linear function or a quadratic function of the integral transform.

In the context of the present disclosure, an integral transform may denote any transform or signal transformation that is adapted to convert a time-dependent signal into a frequency-dependent signal, such as by means of electronic and/or logical operations. The integral transform may involve a continuous integral transform and/or a discrete integral transform.

In the context of the present disclosure, time and frequency may denote any pair of complementary variables.

In an embodiment, the integral transform may comprise a continuous or discrete Fourier transform and/or a fractional Fourier transform and/or a continuous or discrete Radon transform.

The frequency spectrum may be subject to subsequent analysis, in particular with a view to determining distinctions that are characteristic of different physiological states of the human body or animal body to which the first signal and second signal pertain.

To this end, several different frequency spectra may be obtained with the techniques described above, wherein the different frequency spectra differ in their pairs of first signals and second signals from which they are generated. For instance, the different first signals pertaining to different frequency spectra may differ in the times at which they have been acquired, or in the conditions of the human body or animal body from which they have been acquired.

Hence, according to an embodiment the method further comprises comparing the frequency spectrum pertaining to the mixed signal with a reference frequency spectrum, and/or with a second frequency spectrum obtained according to the method with some or all of the features described above.

Employing different integral transforms subsequently or in parallel may also assist the user in better distinguishing between different underlying physiological states. In some embodiments, it may thus make sense to subject the mixtures of the same pairs of first signals and second signals to different integral transforms, and compare the respective frequency spectra with each other, and/or with a reference frequency spectrum.

Hence, according to an embodiment the method may further comprise generating a second frequency spectrum pertaining to the mixed signal.

In particular, generating the second frequency spectrum pertaining to the mixed signal may comprise subjecting the mixed signal to a second integral transform different from the integral transform.

In a second aspect, the disclosure further relates to a computer program or to a computer program product comprising computer readable instructions, such that the instructions, when read on a computer, cause the computer to implement a method with some or all of the features presented above with respect to the first aspect.

In a third aspect, the disclosure relates to a system for converting physiological signals. The system comprises an acquisition unit adapted to obtain a first signal as a function of a time parameter, wherein the first signal represents electrocardiogram data. The acquisition unit is further adapted to obtain a second signal as a function of the time parameter, wherein the second signal represents physiological data different from the electrocardiogram data. The system further comprises a mixing unit adapted to mix the first signal and the second signal to obtain a mixed signal, and a transform unit adapted to generate a frequency spectrum pertaining to the mixed signal.

The system may be adapted to perform a method with some or all of the features presented above with respect to the first aspect.

According to an embodiment, the system comprises a normalization unit adapted to normalize the first signal and/or adapted to normalize the second signal.

According to an embodiment, the mixing unit is adapted to linearly combine the first signal and the second signal, or the normalized first signal and the normalized second signal.

In particular, the first signal and the second signal, or the normalized first signal and the normalized second signal, respectively, may be combined with equal weights of the (normalized) first signal and the (normalized) second signal.

According to an embodiment, the transform unit is adapted to subject the mixed signal to an integral transform to generate the frequency spectrum pertaining to the mixed signal.

The system may further comprise an analysis unit adapted to compare the frequency spectrum pertaining to the mixed signal with a reference frequency spectrum, and/or with a second frequency spectrum.

The acquisition unit and/or the mixing unit and/or the transform unit and/or the normalization unit and/or the analysis unit may each be realized as separate stand-alone units in some embodiments. However, in other embodiments some of the functionality of at least two of these units may be combined into a common unit.

According to an embodiment, the acquisition unit and/or the mixing unit and/or the transform unit and/or the normalization unit and/or the analysis unit may be realized at least partly in hardware.

In other embodiments, the acquisition unit and/or the mixing unit and/or the transform unit and/or the normalization unit and/or the analysis unit may be realized at least partly in software or firmware.

Brief Description of the Figures

The features and numerous advantages of the techniques of the present disclosure will be best apparent from a detailed description of exemplary embodiments with reference to the accompanying drawings, in which:

Fig. 1    is a flow diagram illustrating a method for converting physiological signals according to an embodiment;

Fig. 2    is a schematic illustration of a system for converting physiological signals according to an embodiment;

Fig. 3    is a schematic illustration of a system for converting physiological signals according to another embodiment;

Fig. 4    illustrates the effects of the present disclosure based on experimental data of passive states and active states obtained from a mixture of hydraulic impedance data of the femoral artery and electrocardiogram data in rats; and

Fig. 5    illustrates corresponding experimental data processed by more conventional conversion techniques employing hydraulic impedance data of the femoral artery in the same rats.

Detailed Description

The techniques of the present disclosure will now be described with respect to specific examples of analyzing the hydraulic impedance of blood vessels, such as arteries. However, the same techniques may be employed in the analysis of other physiological data.

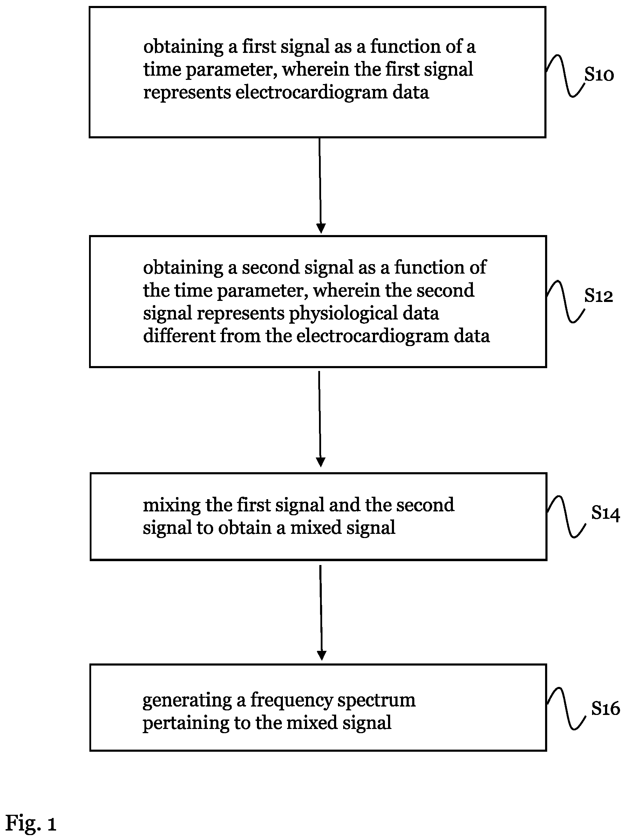

Fig. 1 is a flow diagram illustrating a method for converting physiological signals according to an embodiment.

In a first step Sio, the method involves obtaining a first signal as a function of a time parameter, when the first signal represents electrocardiogram data.

For instance, the first signal $S_1$ may correspond to a time series of signal values $S_1(t_1)$, $S_1(t_2)$, $S_1(t_3)$, ..., $S_1(t_n)$ for a positive integer number $n$, wherein each signal value $S_1(t_i)$, $i = 1,..., n$ represents an electrocardiogram value pertaining to the respective time $t_i$, such as an electrocardiogram value obtained from a pre-acquired or life electrocardiogram of a human or animal body at time $t_i$.

In some embodiments, the first signal may also be represented in terms of a continuous function $S_1(t)$, for some real-valued time parameter $t$, $t_1 \leq t \leq T_1$ within predetermined interval boundaries $t_1$, $T_1$.

Similarly, in a second step S12, a second signal is obtained as a function of the time parameter, wherein the second signal represents physiological data different from the electrocardiogram data.

For instance, the second signal $S_2$ may correspond to a time series of signal values $S_2(t_1)$, $S_2(t_2)$, $S_2(t_3)$, ..., $S_2(t_m)$ for a positive integer number $m$, which may or may not be equal to $n$, wherein each signal value $S_2(t_j)$, $j = 1,..., m$ represents physiological data pertaining to the respective time $t_j$, wherein the physiological data is different from the electrocardiogram data.

The physiological data may be any physiological data pertaining the human body or the animal body, such as an electrical impedance and/or a hydraulic impedance pertaining to a blood vessel of the human body or the animal body. For instance, the electrical impedance of the blood vessel may be obtained from electric resistance measurements along the length of the blood vessel. The hydraulic impedance is known to be functionally related to the electrical impedance. These techniques are generally known in the art, and do not form part of the present disclosure. Similar to the electrocardiogram data of the first signal, each signal value $S_2(t_j)$, $j = 1,..., m$ may represent a pre-acquired or life measurement value of the electrical impedance or hydraulic impedance at time $t_j$.

In some embodiments, the second signal may also be represented in terms of a continuous function $S_2(t)$, for some real-valued time parameter $t$, $t_2 \leq t \leq T_2$ within predetermined interval boundaries $t_2$, $T_2$, which may or may not be equal to the pair of interval boundaries $t_1$, $T_1$.

In a third step S14, the first signal and the second signal are mixed to obtain a mixed signal.

In an example, the mixed signal $M(t)$ may represent a linear combination of the first signal $S_1(t)$ and the second signal $S_2(t)$, in the form

$$M(t) = a\,S_1(t) + b\,S_2(t) + c \qquad (1)$$

with real-valued parameters $a$, $b$, c, wherein $a$ and $b$ are non-zero.

For instance, the mixture may correspond to a weighted average

$$M(t) = p\,S_1(t) + (1-p)\,S_2(t) \qquad (2)$$

of the signals $S_1$ and $S_2$, with a positive weight coefficient $0 < p < 1$.

The mixed signal $M(t)$ may also correspond to sum of the first signal $S_1(t)$ and the second signal $S2(t)$, in the form

$$M(t) = S_1(t) + S_2(t). \qquad (3)$$

In some practically relevant scenarios, the first signal and the second signal may be signals of different dimensions, or may have amplitudes that may vary considerably in strength. It may thus be advantageous to normalize the first signal and/or the second signal prior to the mixing.

For instance, normalizing the first signal $S_1(t)$ may comprise dividing the first signal $S_1(t)$ by a first maximum value $|S_1|$ attained by the first signal over the first time interval $t_1 \leq t \leq T_1$ with predetermined interval boundaries $t_1$, $T_1$. Similarly, normalizing the second signal $S_2(t)$ may comprise dividing the second signal $S_2(t)$ by a second maximum value $|S_2|$ attained by the second signal over the second time interval $t_2 \leq t \leq T_2$ with predetermined interval boundaries $t_2$, $T_2$.

The mixing may then comprise mixing the normalized first signal and the normalized second signal to obtain the mixed signal.

For instance, when taking the normalization into account, the linear mixing according to Eq. (1) reads

$$M(t) = a\,\frac{S_1(t)}{|S_1|} + b\,\frac{S_2(t)}{|S_2|} + c \qquad (4)$$

with the real-valued parameters *a, b, c,* wherein *a* and *b* are non-zero. Similarly, the sum of Eq. (3) reads

$$M(t) = \frac{S_1(t)}{|S_1|} + \frac{S_2(t)}{|S_2|}. \qquad (5)$$

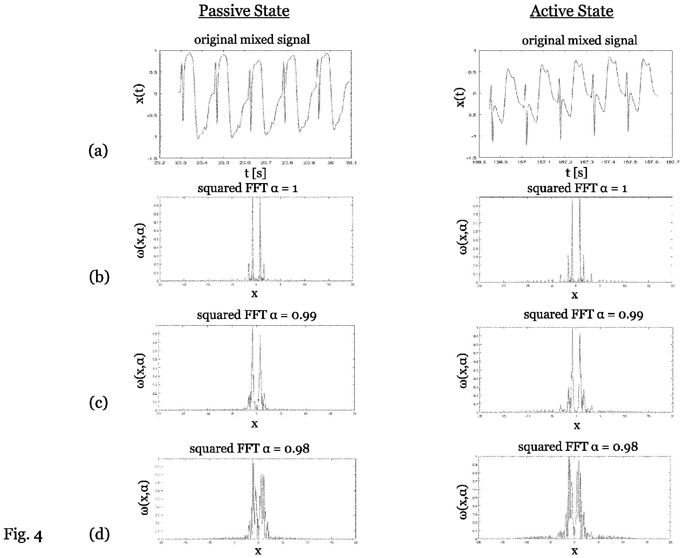

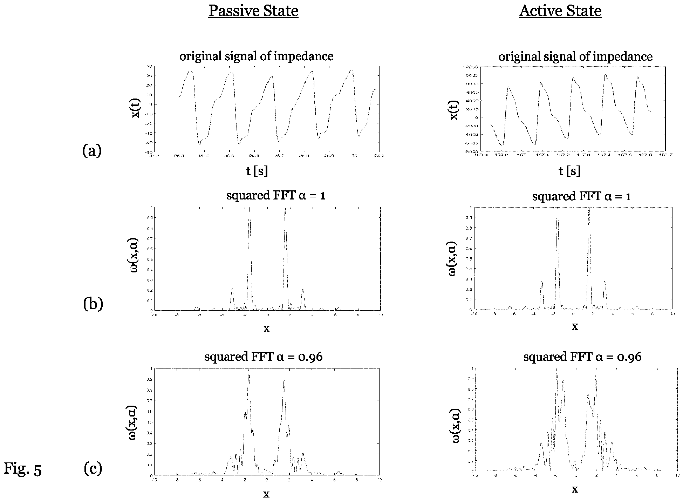

In a fourth step S16, the method involves generating a frequency spectrum pertaining to the mixed signal.
For instance, generating the frequency spectrum may involve applying an integral transform *T* to the mixed signal M(t), in the form

$$M \rightarrow T[M] \qquad (6)$$

to convert the time-dependent mixed signal *M(t)* into a frequency-dependent signal *T[M](ω)*. For instance, the integral transform *T* may involve a Fourier transform or fractional Fourier transform, or a function of the Fourier transform or fractional Fourier transform, such as a square of the Fourier transform or fractional Fourier transform.
The frequency-dependent signal *T[M](ω)* may then be decomposed into individual frequency contributions to analyze the frequency spectrum.
The inventors found that analyzing the spectrum of the mixed signal may provide helpful additional insights into the physiological state of the human or animal body, as compared with an analysis of the spectrum of only the second signal $S_2$ alone, or of only the first signal $S_1$ alone.
Examples of different integral transforms that can be employed in the context of the present disclosure, as well as experimental data that illustrates the effect of analyzing the mixture of the first signal and the second signal as compared to only analyzing the signals individually will be described in further detail below with reference to Figures 4 and 5.
The flow diagram of Fig. 1 shows the method steps S10 to S16 in a certain order. However, this order is for illustration only, and one skilled in the art will appreciate that the order of some of the method steps may be reversed.
For instance, Fig. 1 shows the step S12 of obtaining the second signal after the step S10 of obtaining the first signal. However, in other examples the step of obtaining the second signal may precede the step of obtaining the first signal, or the first signal and the second signal may be obtained (approximately) simultaneously.

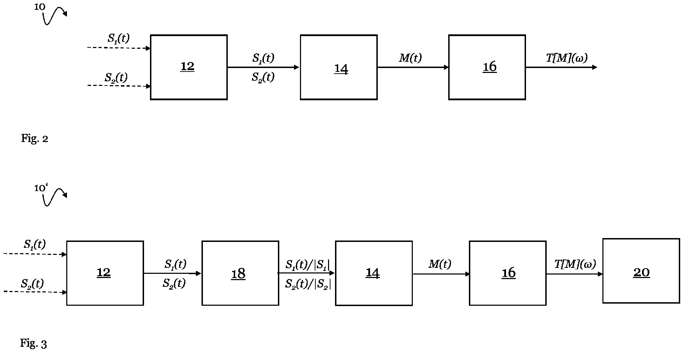

Fig. 2 is a schematic illustration of a corresponding system 10 for converting physiological signals according to an embodiment.
The system 10 comprises an acquisition unit 12, a mixing unit 14 communicatively coupled to the acquisition unit 12, and a transform unit 16 communicatively coupled to the mixing unit 14.
The acquisition unit 12 is adapted to obtain a first signal $S_1(t)$ as a function of a time parameter *t,* wherein the first signal $S_1(t)$ represents electrocardiogram data. The acquisition unit 12 is further adapted to obtain a second signal $S_2(t)$ as a function of the time parameter *t,* wherein the second signal $S_2(t)$ represents physiological data different from the electrocardiogram data.
As illustrated in Fig. 2, the acquisition unit 12 provides the first signal $S_1(t)$ and the second signal $S_2(t)$ to the mixing unit 14, which is adapted to mix the first signal $S_1(t)$ and the second signal $S_2(t)$ to obtain a time-dependent mixed signal *M(t)*. For instance, the mixed signal *M(t)* may represent a sum of the first signal $S_1(t)$ and the second signal $S_2(t)$, in accordance with Eq. (3).
In the embodiment illustrated in Fig. 2, the mixing unit 14 provides the mixed signal *M(t)* to the transform unit 16, and the transform unit 16 may be adapted to generate a frequency spectrum pertaining to the mixed signal *M(t)*. For instance, the transform unit 16 maybe adapted to integral-transform the mixed signal in accordance with Eq. (6), and may decompose the resulting signal *T[M](ω)* into individual frequency contributions that permit a user or computer system to analyze the frequency spectrum.
Fig. 2 shows the acquisition unit 12, the mixing unit 14 and the transform unit 16 as separate and individual units that are communicatively coupled. However, one skilled in the art will understand that this represents just one exemplary configuration, and in other configurations all or some of the acquisition unit 12, the mixing unit 14 and the transform unit 16 may be combined into a single unit.
Fig. 3 is a schematic illustration of another system 10' for converting physiological signals according to an embodiment.

The system 10' shown in Fig. 3 generally corresponds to the system 10 shown in Fig. 2, and corresponding components are denoted with the same reference signs. However, the system 10' additionally comprises a normalization unit 18 in between the acquisition unit 12 and the mixing unit 14, and an analysis unit 20 downstream of the transform unit 16.

The normalization unit 18 is adapted to normalize the first signal $S_1(t)$ and/or the second signal $S_2(t)$. For instance, the normalization unit 18 may be adapted to normalize the first signal $S_1(t)$ by dividing the first signal $S_1(t)$ by a first maximum value $|S_1|$ attained by the first signal $S_1(t)$ over the predetermined first time interval $t_1 \le t \le T_1$. The normalization unit 18 may be further adapted to normalize the second signal $S_2(t)$ by dividing the second signal $S_2(t)$ by a second maximum $|S_2|$ value attained by the second signal $S_2(t)$ over the predetermined second time interval $t_2 \le t \le T_2$.

The normalization unit 18 may then provide both the normalized first signal $S_1(t)/|S_1|$ and the normalized second signal $S_2(t)/|S_2|$ to the mixing unit 14, which may subsequently combine the normalized first signal $S_1(t)/|S_1|$ and the normalized second signal $S_2(t)/|S_2|$ into a time -dependent mixed signal $M(t)$, such as in accordance with Eq. (4) or Eq. (5).

The mixing unit 14 may subsequently provide the time-dependent mixed signal $M(t)$ to the transform unit 16, which may be adapted to generate a frequency spectrum pertaining to the mixed signal $M(t)$, as described above with reference to Fig. 2.

The analysis unit 20 may be adapted to receive the frequency spectrum from the transform unit 16, and may be further adapted to compare the frequency spectrum pertaining to the mixed signal $M(t)$ with a reference frequency spectrum, and/or with a second frequency spectrum.

For instance, the reference frequency spectrum may represent a previously acquired spectrum representing reference date of the human or animal body. By comparing the frequency spectrum pertaining to the mixed signal $M(t)$ with the reference spectrum, such as by means of automated data analysis, distinctions may be identified that could be used to later classify the acquired frequency spectrum pertaining to the mixed signal $M(t)$. These techniques may provide helpful insights into the current physiological state of the human or animal body.

Alternatively or additionally, the analysis unit 20 may be adapted to compare different frequency spectra generated according to the techniques described above with reference to Figures 1 to 3, but generated by means of a plurality of different integral transforms T, T'. A comparison of the corresponding frequency spectra of the frequency-dependent signals $T[M](\omega)$, $T'[M](\omega)$ may allow to determine the integral transform that is most useful for distinguishing between different physiological states of the human or animal body, in a given scenario.

Some exemplary integral transforms $T[M]$ that maybe employed in the context of the present disclosure will now be described in additional detail.

The Wigner function and the tomographic representation

One can assume that the totality of all signal values is represented by a set of vectors $|f\rangle$ in the space of all possible values. Symbol $f$ designates a signal amplitude in this case. This value can be a complex one in general. The space of states should be complete, i.e.,

$$\langle f'|f \rangle = \delta(f - f') \quad and \quad \int \mathrm{d}f |f\rangle\langle f| = \hat{1} . \tag{7}$$

This assumes that a signal amplitude can take continuous values in some interval $[f_{min}, f_{max}]$. In other words, it has a continuous spectrum. A signal takes discrete values if it is represented in a digital form. In this case properties (7) can be rewritten as follows

$$\langle f'|f \rangle = \delta_{f,f'} \quad and \quad \sum_{f=f_{min}}^{f_{max}} |f\rangle\langle f| = \hat{1}. \tag{8}$$

Let us introduce a density matrix $\rho^s$ of a signal using quantum mechanics methods. It can be represented as a density matrix of a pure state in the form:

$$\rho^s = |f\rangle\langle f|. \tag{9}$$

This relation in a "time-representation" has the following expression:

$$\rho^s(t,t') = \langle t|f\rangle\langle f|t'\rangle \equiv f(t)f^*(t'). \tag{10}$$

Let us define the Wigner-Ville function of a time-dependent signal $\rho^s(t)$ as follows:

$$W^s(\omega, t) = \int_{-\infty}^{+\infty} dx\, \rho^s\left(t + \frac{x}{2}, t - \frac{x}{2}\right) e^{-i\omega x}.$$ (11)

The reverse transform is determined in complete correspondence with the Wigner function of a quantum system:

$$\rho^s(t, t') = \int_{-\infty}^{+\infty} \frac{d\omega}{2\pi} W^s\left(\omega, \frac{t+t'}{2}\right) e^{i\omega(t-t')}.$$ (12)

A signal can be expressed through the Wigner function using Eq. (10) for $t' = 0$:

$$f(t)f^*(0) = \int_{-\infty}^{+\infty} \frac{d\omega}{2\pi} W^s\left(\omega, \frac{t}{2}\right) e^{i\omega t}.$$ (13)

The value of a signal at the initial time in any case can be represented as follows:

$$f^*(0) = |f(0)|\, e^{-i\varphi(0)},$$ (14)

where a phase $\varphi(0)$ at the initial time remains undetermined. Accordingly, we have

$$f(t) = e^{i\varphi(0)} \frac{\frac{1}{2\pi}\int_{-\infty}^{+\infty} d\omega\, W^s\left(\omega, \frac{t}{2}\right) e^{i\omega t}}{\sqrt{\frac{1}{2\pi}\int_{-\infty}^{+\infty} d\omega\, W^s(\omega, 0)}}.$$ (15)

Now we can define a signal tomogram

$$w^s(X, \mu, \nu) = \iiint_{-\infty}^{\infty} \frac{dt\, d\omega\, du}{(2\pi)^2} W^s(\omega, t)\, e^{-iu(X - \mu t - \nu\omega)}$$ (16)

Here $X$ is an eigen value of a Hermitian operator

$$\hat{X} = \mu\hat{t} + \nu\hat{\omega}, \quad \mu^2 + \nu^2 = 1.$$ (17)

One takes as usual

$$\mu = \cos\theta, \nu = \sin\theta, \quad 0 \le \theta \le \pi/2.$$ (18)

The eigen value of $\hat{X}$ is located on the straight line of the first quadrant in the plane of variables $(t, \omega)$. The Wigner function can be written down explicitly through a signal function after substituting Eq. (16) into Eq. (12),

$$w^s(X, \mu, \nu) = \int \int \int \int \frac{dt\, d\omega\, du\, dy}{(2\pi)^2} f\left(t + \frac{y}{2}\right) f^*\left(t - \frac{y}{2}\right) e^{-i\omega y} e^{-iu(X - \mu t - \nu\omega)}.$$ (19)

One can integrate Eq. (19) over the $\omega$ variable

$$\int_{-\infty}^{\infty} d\omega\, e^{-i\omega(y - \nu u)} = 2\pi\delta(y - \nu u).$$ (20)

Thus we have

$$w^s(X, \mu, v) = \frac{1}{2\pi} \iiint \mathrm{d}t\mathrm{d}u\mathrm{d}y\, f(t + y/2)f^*(t - y/2)\, \mathrm{e}^{-iu(X-\mu t)}\delta(y - vu). \qquad (21)$$

Performing the integration over $u$ we obtain

$$w^s(X, \mu, v) = \frac{1}{2\pi|v|} \iint \mathrm{d}t\mathrm{d}y\, f\left(t + \frac{y}{2}\right) f^*\left(t - \frac{y}{2}\right) \mathrm{e}^{-\mathrm{i}\frac{y}{v}(X-\mu t)}. \qquad (22)$$

Let us change variables in the integral of Eq. (22) as follows:

$$t = (z + q)/2, \quad y = z - q. \qquad (23)$$

The Jacobian of the transform of Eq. (23) is equal to -1, but the integration limits extend from $-\infty$ to $+\infty$ and we obtain

$$w^s(X, \mu, v) = \frac{1}{2\pi|v|} \iint \mathrm{d}z\mathrm{d}q\, f(z)f^*(q)\, \mathrm{e}^{-\mathrm{i}\frac{1}{v}((z-q)X-\mu(z^2-q^2)/2)} =$$

$$= \frac{1}{2\pi|v|} \left| \int_{-\infty}^{\infty} \mathrm{d}z f(z) \mathrm{e}^{\frac{\mathrm{i}\mu}{2v}z^2 - \frac{\mathrm{i}Xz}{v}} \right|^2. \qquad (24)$$

Finally, let us rewrite a tomogram definition introducing a more conventional designation of the integration variable by letter $t$ as follows:

$$w^s(X, \mu, v) = \frac{1}{2\pi|v|} \left| \int_{-\infty}^{\infty} f(t) \exp\left[\frac{\mathrm{i}\mu}{2v}t^2 - \frac{\mathrm{i}Xt}{v}\right] \mathrm{d}t \right|^2. \qquad (25)$$

The obtained tomographic representation is connected with the so-called fractional Fourier transform (FRFT), which is widely used for example in quantum optics. On the other hand, it corresponds to the so-called Radon transform. As usual, the FRFT can be written in the form

$$F(X, \alpha) = \sqrt{\frac{v - \mathrm{i}\mu}{2\pi v}} \exp\left(\frac{\mathrm{i}\mu}{2v}X^2\right) \int_{-\infty}^{\infty} \exp\left[\frac{\mathrm{i}}{2v}(-2Xt + \mu t^2)\right] f(t)\mathrm{d}t. \qquad (26)$$

The dimensionless variable $\alpha$ may vary in the interval $0 \le \alpha \le 1$ and may be called the transform order. The transform order is functionally connected with the previously introduced parameters $\theta$, $\mu$ and $v$ by the relation $\alpha = 2\theta/\pi$. In this case relation (18) can be rewritten a follows:

$$\mu = \cos\frac{\pi}{2}\alpha, \quad v = \sin\frac{\pi}{2}\alpha \qquad (27)$$

It is then easy to see that the tomographic representation is equal to the square of the FRFT modulus,

$$w^s(X, \mu, v) = |F(X, \alpha)|^2 \qquad (28)$$

Tomogram calculation

The identical transform is realized at $\alpha = 0$ and at $\alpha = 1$ we obtain the Fourier transform. Numerical calculations of this kind of transform on a computer may be complicated in practice by two reasons. Firstly, the real signal (cardiogram) has a complex fine-scale structure and demands large numbers of grid nodes in a numerical representation. Secondly, the integral transform core in Eq. (26) is an imaginary exponent of a quadratic function. So, it is a frequently oscillating function and gives additional problems in approximation of the integral by quadrature formulae. One way to overcome these difficulties is a reduction of the FRFT to an integral of a convolution type. The following application of the fast Fourier transform algorithm makes the procedure more successful. Using the identity

$$\mu X^2 - 2Xt + \mu t^2 = (\mu - 1)X^2 + (X - t)^2 + (\mu - 1)t^2 \tag{29}$$

as presented by H. M. Ozaktas et al., IEEE Transactions on Signal Processing 44 (1996) 2141, one can transform Eq. (26) into the following form:

$$F(X, \alpha) = \sqrt{\frac{\nu - i\mu}{2\pi\nu}} \exp\left(\frac{i(\mu-1)}{2\nu} X^2\right) \int_{-\infty}^{\infty} \exp\left[\frac{i}{2\nu}(X - t)^2\right] \tilde{f}(t)\, dt \tag{30}$$

with

$$\tilde{f}(t) = \left(\frac{i(\mu-1)}{2\nu} t^2\right) f(t). \tag{31}$$

The integral in Eq. (30) has a type of a convolution.

Generally, the transform order $\alpha$ may be chosen in the range $0 \leq \alpha \leq 1$, in particular $\alpha < 1$. In some embodiments, the transform order $\alpha$ may be chosen no smaller than 0.90, in particular no smaller than 0.95.

In exemplary numerical calculations, the interval $0 \leq \alpha \leq 1$ can be broken into 50 equal distances, and the transform order $\alpha$ can thus take 51 values including the boundaries of the interval. The quadrature formula for integral (30) may be constructed on a uniform grid. The grid size may be determined automatically for the given precision of the calculations. A permissible relative error of calculations maybe taken to be of the order of $10^{-5}$. This precision may be achieved with a grid size up to $2^{24}$ knots. One tomogram calculation on a PC may be executed from few to tens of minutes of computer time, due to the Fast Fourier Transform (FFT) algorithm application.

It should be noted that a tomogram form essentially depends on a choice of a unit of time representation. In primary data received from a device, the time $T$ may be given in seconds as usual. When a tomogram is calculated, a dimensionless time $t = T/T_0$ may be used, where $T_0$ may be a unit of time in seconds. A frequency scale in the tomogram cross-section at $\alpha = 1$ may be determined unambiguously by a choice of $T_0$. Namely, a value $\omega = T_0^{-1} X$ may be a circular frequency, and it may have a dimension of rad/sec.

To illustrate the techniques of the present disclosure, Fig. 4 shows an example of the original time series of the sum of normalized electrocardiogram data and the normalized hydraulic impedance of the femoral artery in rats determined according to Eq. (5) above (Fig. 4a), the corresponding squared Fourier spectrum (Fig. 4b) and two tomograms for different transform orders $\alpha = 0.99$ (Fig. 4c) and $\alpha = 0.98$ (Fig. 4d), for two different states of the artery (passive state and active state). The hydraulic impedance has been determined by means of electrical resistance measurements along the length of the femoral artery.

In the example of the frequency spectra shown in Fig. 4, in the passive state only two harmonics have a large amplitude, whereas the third harmonic is minimum or is equal to 0. This effect is revealed already in the squared fractional Fourier transform according to Fig. 4b. The distinctions are even more pronounced in the tomograms according to Figures 4c and 4d. The amplitudes of the small frequencies in the active state exceed the corresponding amplitudes of the small frequencies in the passive state by a factor of 5 to 7.

For comparison, Fig. 5 shows a corresponding example of the original time series of the hydraulic impedance only (Fig. 5a), without the mixture, the corresponding squared Fourier spectrum (Fig. 5b) and a tomogram (Fig. 5c) with transform order $\alpha = 0.96$ of the femoral artery in rats, again for two different states of the artery (passive state and active state).

A comparison reveals that the squared fractional Fourier transforms according to Fig. 5b are largely identical for the passive state and the active state, and the tomograms according to Fig. 5c likewise fail to provide a clear trend that permits to uniquely distinguish between the passive state and the active state.

The description of the embodiments and the figures merely serve to illustrate the techniques of the present disclosure and the beneficial effects associated therewith, but should not be understood to imply any limitation. The scope of the disclosure is to be determined from the appended claims.

Reference Signs

| 10, 10' | system for converting physiological signals |
| 12 | acquisition unit |
| 14 | mixing unit |
| 16 | transform unit |
| 18 | normalization unit |
| 20 | analysis unit |

**Claims**

1. A method for converting physiological signals, comprising:

    obtaining a first signal ($S_1$) as a function of a time parameter, wherein the first signal ($S_1$) represents electrocardiogram data;
    obtaining a second signal ($S_2$) as a function of the time parameter, wherein the second signal ($S_2$) represents physiological data different from the electrocardiogram data;
    mixing the first signal ($S_1$) and the second signal ($S_2$) to obtain a mixed signal (M); and
    generating a frequency spectrum pertaining to the mixed signal (M).

2. The method according to claim 1, wherein the physiological data comprises rheogram data, in particular rheogram data of a blood vessel.

3. The method according to claim 1 or 2, further comprising:

    normalizing the first signal ($S_1$) and/or normalizing the second signal ($S_2$), prior to mixing the first signal ($S_1$) and the second signal ($S_2$).

4. The method according to claim 3, wherein:

    normalizing the first signal ($S_1$) comprises dividing the first signal ($S_1$) by a first maximum value attained by the first signal ($S_1$) over a predetermined first time interval; and/or
    normalizing the second signal ($S_2$) comprises dividing the second signal ($S_2$) by a second maximum value attained by the second signal ($S_2$) over a predetermined second time interval.

5. The method according to any of the preceding claims, wherein mixing the first signal ($S_1$) and the second signal ($S_2$) comprises linearly combining the first signal ($S_1$) and the second signal ($S_2$), in particular with equal weights of the first signal ($S_1$) and the second signal ($S_2$).

6. The method according to any of the preceding claims, wherein the mixed signal (M) is given in terms of a sum of the first signal ($S_1$) and the second signal ($S_2$).

7. The method according to any of the preceding claims, wherein generating the frequency spectrum pertaining to the mixed signal (M) comprises subjecting the mixed signal (M) to an integral transform.

8. The method according to claim 7, wherein the integral transform comprises a Fourier transform and/or a fractional Fourier transform and/or a Radon transform.

9. The method according to any of the preceding claims, further comprising:

    comparing the frequency spectrum pertaining to the mixed signal (M) with a reference frequency spectrum, and/or with a second frequency spectrum obtained according to the method according to any of the preceding claims.

10. A computer program comprising computer-readable instructions, such that the computer-readable instructions, when read on a computer, cause the computer to implement a method according to any of the preceding claims.

11. A system (10, 10') for converting physiological signals, comprising:

an acquisition unit (12) adapted to obtain a first signal ($S_1$) as a function of a time parameter, wherein the first signal ($S_1$) represents electrocardiogram data; and adapted to obtain a second signal ($S_2$) as a function of the time parameter, wherein the second signal ($S_2$) represents physiological data different from the electrocardiogram data;
a mixing unit (14) adapted to mix the first signal ($S_1$) and the second signal ($S_2$) to obtain a mixed signal (M); and
a transform unit (16) adapted to generate a frequency spectrum pertaining to the mixed signal (M).

12. The system (10, 10') according to claim 11, further comprising:

a normalization unit (18) adapted to normalize the first signal ($S_1$) and/or adapted to normalize the second signal ($S_2$).

13. The system (10, 10') according to claim 11 or 12, wherein the mixing unit (14) is adapted to linearly combine the first signal ($S_1$) and the second signal ($S_2$), in particular with equal weights of the first signal ($S_1$) and the second signal ($S_2$).

14. The system (10, 10') according to any of the claims 11 to 13, wherein the transform unit (16) is adapted to subject the mixed signal (M) to an integral transform to generate the frequency spectrum pertaining to the mixed signal (M).

15. The system (10, 10') according to any of the claims 11 to 14, further comprising:

an analysis unit (20) adapted to compare the frequency spectrum pertaining to the mixed signal (M) with a reference frequency spectrum, and/or with a second frequency spectrum.

obtaining a first signal as a function of a
time parameter, wherein the first signal
represents electrocardiogram data

S10

obtaining a second signal as a function of
the time parameter, wherein the second
signal represents physiological data
different from the electrocardiogram data

S12

mixing the first signal and the second
signal to obtain a mixed signal

S14

generating a frequency spectrum
pertaining to the mixed signal

S16

Fig. 1

$S_1(t)$

$S_2(t)$

| | |
|---|---|
| **12** | |

$S_1(t)$
$S_2(t)$

| | |
|---|---|
| **14** | |

$M(t)$

| | |
|---|---|
| **16** | |

$T[M](\omega)$

10

Fig. 2

$S_1(t)$

$S_2(t)$

| | |
|---|---|
| **12** | |

$S_1(t)$
$S_2(t)$

| | |
|---|---|
| **18** | |

$S_1(t)/|S_1|$
$S_2(t)/|S_2|$

| | |
|---|---|
| **14** | |

$M(t)$

| | |
|---|---|
| **16** | |

$T[M](\omega)$

| | |
|---|---|
| **20** | |

10'

Fig. 3

Fig. 4

Passive State      Active State

(a) original mixed signal / original mixed signal
(b) squared FFT α = 1 / squared FFT α = 1
(c) squared FFT α = 0.99 / squared FFT α = 0.99
(d) squared FFT α = 0.98 / squared FFT α = 0.98

Passive State                                              Active State

(a)

(b)

Fig. 5     (c)

Fig. 5

# EP 4 115 799 A1

Europäisches Patentamt

European Patent Office

Office européen des brevets

## EUROPEAN SEARCH REPORT

Application Number

EP 21 18 4845

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ODINAKA IKENNA ET AL: "Cardiovascular Biometrics: Combining Mechanical and Electrical Signals", IEEE TRANSACTIONS ON INFORMATION FORENSICS AND SECURITY, IEEE, USA, vol. 10, no. 1, 1 January 2015 (2015-01-01), pages 16-27, XP011565633, ISSN: 1556-6013, DOI: 10.1109/TIFS.2014.2361261 [retrieved on 2014-12-03] * pages 17-19 * * pages 25-26 * | 1-15 | INV. A61B5/0205 A61B5/053 A61B5/0535 A61B5/02 A61B5/347 A61B5/00 A61N1/37 A61B5/346 |
| X | WO 2014/123512 A1 (VIVAQUANT LLC [US]) 14 August 2014 (2014-08-14) * page 9, line 8 - page 10, line 6 * * page 26, lines 14-23; claim 14; figures 21,25 * | 1,2,11, 12 | |
| A | KLYPIN DMITRY N ET AL: "The base tasks in development of Biomedical Data Information System", 2016 DYNAMICS OF SYSTEMS, MECHANISMS AND MACHINES (DYNAMICS), IEEE, 15 November 2016 (2016-11-15), pages 1-4, XP033044346, DOI: 10.1109/DYNAMICS.2016.7819027 [retrieved on 2017-01-13] * the whole document * | 1-15 | TECHNICAL FIELDS SEARCHED (IPC)  A61B A61N |
| A | WO 2011/080194 A1 (GAMBRO LUNDIA AB [SE]; OLDE BO [SE]; SOLEM KRISTIAN [SE]) 7 July 2011 (2011-07-07) * page 50, line 4 - page 51, line 36; claim 1; figures 7-10 * | 1-15 | |

-/--

The present search report has been drawn up for all claims

| Place of search | Date of completion of the search | Examiner |
|---|---|---|
| Munich | 6 December 2021 | Gentil, Tamara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

European Patent Office

Europäisches
Patentamt
European
Patent Office
Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/125376 A1 (DENNEY JR THOMAS S [US] ET AL) 10 May 2018 (2018-05-10) * the whole document * ----- | 1-15 | |

TECHNICAL FIELDS
SEARCHED        (IPC)

The present search report has been drawn up for all claims

| Place of search | Date of completion of the search | Examiner |
|---|---|---|
| **Munich** | **6 December 2021** | **Gentil, Tamara** |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 4 115 799 A1**

## ANNEX TO THE EUROPEAN SEARCH REPORT
## ON EUROPEAN PATENT APPLICATION NO.

EP 21 18 4845

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2021

| Patent document cited in search report | | | Publication date | Patent family member(s) | | | Publication date |
|---|---|---|---|---|---|---|---|
| WO 2014123512 | A1 | | 14-08-2014 | AU | 2013377909 | A1 | 20-08-2015 |
| | | | | CA | 2900160 | A1 | 14-08-2014 |
| | | | | EP | 2953533 | A1 | 16-12-2015 |
| | | | | WO | 2014123512 | A1 | 14-08-2014 |
| WO 2011080194 | A1 | | 07-07-2011 | CN | 102740902 | A | 17-10-2012 |
| | | | | EP | 2519279 | A1 | 07-11-2012 |
| | | | | ES | 2541611 | T3 | 22-07-2015 |
| | | | | US | 2013006130 | A1 | 03-01-2013 |
| | | | | WO | 2011080194 | A1 | 07-07-2011 |
| US 2018125376 | A1 | | 10-05-2018 | CA | 3082253 | A1 | 17-05-2018 |
| | | | | CN | 110198664 | A | 03-09-2019 |
| | | | | EP | 3537962 | A1 | 18-09-2019 |
| | | | | JP | 6888086 | B2 | 16-06-2021 |
| | | | | JP | 2020501630 | A | 23-01-2020 |
| | | | | US | 2018125376 | A1 | 10-05-2018 |
| | | | | WO | 2018089720 | A1 | 17-05-2018 |

EPO FORM P0459

For more details about this annex : see Official Journal of the European Patent Office, No. 12/82

19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Y. M. BELOUSOV et al.** Tomographic representation of ECG signal. *J. Russ. Laser Res.,* 2018, vol. 39 (3

- **H. M. OZAKTAS et al.** *IEEE Transactions on Signal Processing,* 1996, vol. 44, 2141